# EUROPEAN PATENT APPLICATION

(11) **EP 0 724 892 A1**
(43) Date of publication of application: **07.08.1996**
(21) Application number: 95929264.0
(22) Date of filing: 12.07.1995
(51) Int. Cl.: A61N 1/16

(54) **METHOD OF PROTECTING A LIVING ORGANISM USING ENERGY AND A DEVICE FOR CARRYING OUT SAID METHOD**

(30) Priority: 28.07.1994 RU 94028406; 23.01.1995 RU 95100856; 19.04.1995 RU 95106024
(71) Applicant: Belov, Dmitry Viktorovich, Moscow, 129346 (RU)
(72) Inventor: BELOV, Dmitry Viktorovich, Moscow, 129346 (RU); BUINY, Sergei Leonidovich, Tula, 300034 (RU); BURTSEVA, Irina Konstantinovna, Tula, 300045 (RU); POLUBESOV, Gennady Sergeevich, Tula, 300045 (RU); POTAPOV, Alexei Anatolievich, Moscow, 121614 (RU)
(74) Representative: W.P. Thompson & Co.
(86) International application number: RU9500151
(87) International publication number: WO9603178

(57) **Abstract**

The claimed method involves the creation of an energy field (28) which has the effect of an ellipsoidal bioenergy screen and is allowed to act on the living organism (27). The energy field (28) is recorded via an optical communications line on a data carrier (30) after being converted into electromagnetic waves. The recorded information can be reproduced repeatedly to act on the organism (27). The proposed device comprises a hollow sealed housing (1) made of a non-magnetic material with an earth contact (2). Inside the housing (1) is a system for creating a high-frequency arc discharge and a resonant L-C network (3).

## Description

### Technical Field

The present invention relates in general to medical engineering made use of in non-traditional medicine and has particular reference to a method and a device for energy protection of a biological object.

### Background Art

The problem how to protect the objects of animate nature, human beings in particular, against various factors affecting adversely the vital activity of living organisms becomes very urgent at the present time distinguished for a general deterioration of the ecological situation. To such factors may be attributed a variety of radiations, diverse geopathogenic zones, wherein for various reasons a biological object may be found, as well as unauthorized extrasensory effects.

Known in the prior art is a method for radiation protection (DE, A1, 3,407,319), whereby a shielding field is built up nearby the radiation source so as to prevent a radiation harmful to a living organism to penetrate thereto.

Known in the present state of the art is a radiation protection device (DE, A1, 3,407,319), which is essentially a non-magnetic casing having a grounding terminal and accommodating a conductor connected to said grounding terminal.

The aforementioned known method and device are instrumental in establishing a shielding energy field in a small space but fail to afford protection against unauthorized extrasensory effects.

Known in the art presently is also a method for energy protection of a biological object (DE, A1, 3,525,521), consisting in that there is established a shielding energy field concentrated in a confined space, and a biological object is exposed to the effect of said field.

One more prior-art method for energy protection of a biological object is known to use presently (cf. "Electric puncture diagnosis, homeopathy, and the phenomenon of telekinesis" by N.L.Lupichev, Research and Production Company "Irius", Moscow 1990, pp.40-42 (in Russian), consisting in that an energy field is created and connected, through an optical transmission line, to a recorder, whereupon the information thus recorded on an information medium is transmitted for a biological object to be exposed to the effect thereof.

The aforesaid known method is instrumental in recording, through an optical transmission line, the energy and information properties of said field on a material information medium, which thus acquires itself the properties of an irradiator, and in using said properties for producing effects on a biological object, that is, to provide an indirect effect which is much weaker compared to the source of the energy field (i.e., the irradiator).

One more device for energy protection of a biological object (DE, A1, 3,525,521) is known to comprise a non-magnetic housing appearing as a plane-base geometrical figure in the form of a pyramidal body having a hollow in the central part thereof and oriented with one of its edges towards the north magnetic pole of the Earth.

It is due to the pyramidal shape of its housing and its predetermined orientation that a specific magnetic energy field is established inside the housing, which contributes to elimination of energy dysfunctions in the organism of a biological object, human beings in particular, and is capable of forming a kind of biological shield affording protection against harmful radiations.

However, the device discussed before uses only the directivity of the Earth's magnetic field, while energy is accumulated in the inner hollow of the pyramidal body, namely, in a strictly definite place as for height thereof. Concentration of said energy field depends on the angle of inclination of the planes of sides of the pyramidal body, the number and dimensions of said sides.

The known device discussed before is used, as a rule, for rendering a protective (therapeutic) effect to the appropriate parts of human organism, since to place the whole human body to that space of the pyramidal body wherein a maximum energy field concentration occurs, is a fairly difficult task. This is due to considerable geometrical dimensions of the device, which hampers its operation and makes it operable only under stationary conditions.

Additionally, to control over the effect produced by an energy field on a biological object is likewise a fairly hard task to perform.

### Disclosure of the Invention

It is a principal object of the invention to provide a method and a device for energy protection of a biological object, which method and device are instrumental in providing a possibility of recording and transferring a shielding-energy field concentrated outside the device and affording a protective effect against both harmful radiations and unauthorized extrasensory effects by exposing the biological object both to the direct effect of an energy field and to that recorded on an information medium so as to substantially extend the functional capability of the proposed method and device, all this being attainable due to the provision of an energy field featuring appropriate shape, orientation, and properties, conversion of said energy field, construction arrangement of the housing of said device, and equipping the device with appropriate additional units.

The foregoing object is accomplished due to the fact that in a method for energy protection of a biological object residing in that an energy field is established, concentrated in a confined space, and a biological object is exposed to the effect of said energy field, according to the invention, the energy field possesses the properties of a biological-energy shield and is shaped as an ellipsoidal solid of revolution having its major axis vertically oriented.

The foregoing object is accomplished also due to the fact that in a method for energy protection of a biological object residing in that an energy field is established and transmitted, through an optical transmission line, to a recorder, whereupon the information thus recorded on an information medium is transmitted for a biological object to be exposed to the effect thereof, according to the invention, the energy field possesses the properties of a biological energy shield in the form of an ellipsoidal solid of revolution having its major axis oriented vertically, and recording said energy field is preceded by converting it into electromagnetic waves which are then transmitted along electromagnetic communication lines to an information medium with a possibility of repeated reproduction of said information for a biological object to be exposed to the effect thereof, the shape and properties of the reproduced energy field being similar to those of the originally built up energy field.

It is expedient that information in the form of electromagnetic waves is transmitted to at least one receiver.

It is favorable that reproduction of said information is preceded by its transcribing to a similar or different information medium.

The herein-proposed method allows of preliminarily producing an image of the biological object and exposing said image to the effect of the reproduced information, thus producing the effect of said information directly on the biological object.

It is practicable, according to the method proposed herein, that the image of the biological object is created in the form of electromagnetic waves which are then transmitted to one of the receivers, or as a photograph.

It is convenient that when reproducing the information, the intensity of the effect produced by the energy field on the image of the biological object is chosen such that a direct relationship of said intensity to the ratio between the actual size of the image of the biological object and the actual dimensions of the energy field is observed.

It is likewise expedient that when reproducing the information, the intensity of the effect produced by the energy field on the image of the biological object is chosen such that there is a direct relationship of said intensity to the period of time that has elapsed from the instant when the image of the biological object represented on the receiver is produced.

The foregoing object is accomplished also due to the fact that a device for energy protection of a biological object, comprising a non-magnetic hollow housing appearing as a plane-base geometrical figure, according to the invention, comprises a means for producing a high-frequency arc discharge, said means being accommodated in said housing which is closed and is provided with a grounding terminal.

It is expedient that the means for producing a high-frequency arc discharge comprises a high-voltage power source, a low-frequency generator, a high-frequency arc-discharge generator, and a resonant LC-circuit, all of them being parallel-connected to one another.

It is practicable that the high-frequency arc-discharge generator is built around a gas-discharge tube having coaxial electrodes.

It is reasonable that the device comprises a capacitor and an inductor building up an additional LC-circuit connected to the housing and the grounding terminal at a single point whose position is chosen such that a maximum loop of the energy field electromagnetic component be over the external surface of the housing.

It is preferable that the housing is made from aluminium and is cube-shaped.

It is convenient that the means for producing a high-frequency arc discharge is enclosed in the housing so that a distance to its geometrical center is variable.

To provide a possibility of information recording and transferring, it is effective that the device is equipped with a video camera so positioned as to perform video-filming of the generated energy field, and with at least one television receiver and a means for reproducing the recorded information on the generated energy field.

The invention proposed herein is instrumental in producing a stable shielding energy field having an increased degree of effect and makes it possible to control the intensity of the produced effect irrespective of the orientation of the device, the shape and directivity of the field remaining invariable. As a matter of fact, there is established a kind of biological-energy shield within which protection is afforded to a biological object against a variety of radiations adversely affecting its energetics, to which may be attributed the effect of geopathogenic zones, unauthorized extrasensory effects, and other factors. Furthermore, the zone of action of the shielding energy field provides for normalization of metabolic processes, enhances immunity and hence renders a positive effect to the general state of the organism as a whole by mobilizing organism's own intrinsic forces, which are no longer suppressed by the effect of harmful energy, for struggle against diverse diseases.

According to the method proposed herein, there is provided a possibility of recording and transferring the thus-produced shielding energy field, through optical transmission lines, to an information medium with possible subsequent many-times repeated reproduction in one or more receivers for exposing a biological object to the effect of said information. The protective properties of the recorded field are fully retained

An information about the shielding energy field can be transcribed to a similar or different information medium.

A device for energy protection of biological objects is featured by a simple construction, is realized on the grounds of an extensively known elementary base, and can readily be productionized. Moreover, the device is simple in handling and needs no special skills.

According to one of the embodiments of the present invention, provision is therein made for recording the energy field onto an information medium with a possibility of subsequent many-times repeated reproduction. To this aim, the device is provided with a video camera and a television receiver on the screen of which the recorded information is reproduced, possessing the same protective properties as the energy field itself.

Thus, the method and device of the present invention provides, when producing an immediate effect on biological objects and when such an effect is applied in the form of a recording, a therapeutic effect by relieving stress states, getting locations rid of "foul" energy resultant, e.g., from radiation injury, protection against unauthorized extrasensory effects, and other factors.

The fact that the present invention can be realized using readily available technical means made use of extensively under domestic conditions, renders it widely applicable.

### Brief Description of the Drawings

In what follows the present invention is illustrated in more detail by description of some specific exemplary embodiments thereof, an example, and the accompanying drawings, wherein:
FIG.1 is a general view of a device for energy protection of a biological object, showing its housing partly cut away, according to the invention;
FIG.2 is a schematic electric circuit diagram of a means for producing a high-frequency arc discharge, according to the invention;
FIG.3 is a view of FIG.1, showing an additional resonant LC-circuit, according to the invention;
FIG.4 is a view of FIG.1, showing a mobile means for producing a high-frequency arc discharge, according to the invention;
FIG.5 is a view of an energy field, according to the invention;
FIG.6 is a time chart of advancing arc-discharge high-frequency pulses; and
FIG.7 is a functional diagram of the proposed device provided with a video camera and a television receiver, according to the invention.

### Best Method of Carrying Out the Invention

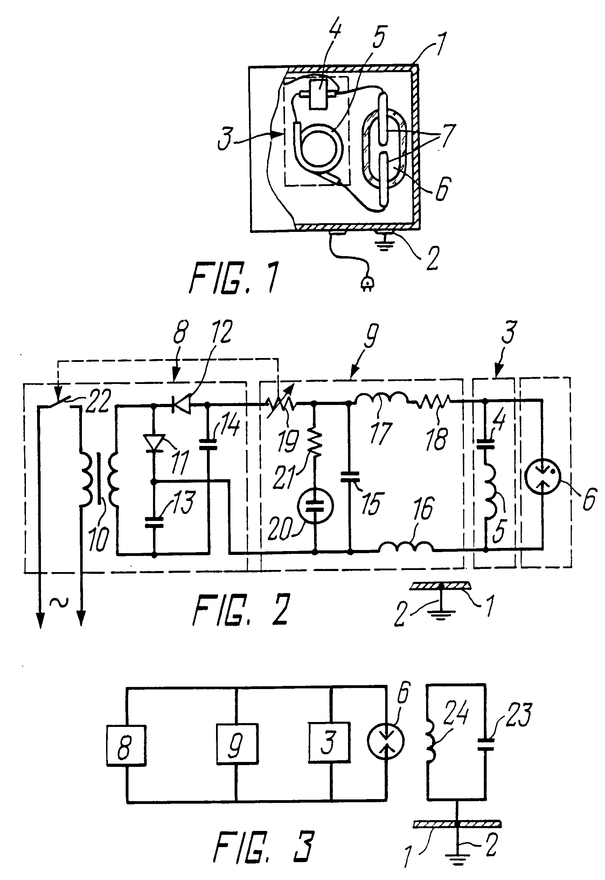

The device for energy protection of a biological object, according to the invention, comprises a non-magnetic housing 1 (FIG.1) shaped as a plane-base geometrical figure and provided with a grounding terminal 2.

In the general case the housing 1 may be a prism, a pyramid, a cylinder, a cone, a cone frustum, portion of a sphere, and so on. In this particular embodiment the housing 1 is cube-shaped and is made from aluminium.

The housing 1 accommodates a means for producing a high-frequency arc discharge, comprising a resonant LC-circuit 3, consisting of series-connected a capacitor 4 and an inductor 5, and a high-frequency arc-discharge generator built around a gas-discharge tube 6 having coaxial electrodes 7 connected to the LC-circuit 3.

In addition, the means for producing a high-frequency arc discharge comprises a high-voltage power source 8 (FIG.2) having leads for connection to A.C. supply mains, and a low-frequency generator 9, the inputs of said generator and of the gas-discharge tube 6 being connected to the outputs of the power supply source 8.

In a particular case the D.C. high-voltage power source 8 consists of a transformer 10, diodes 11 and 12, and capacitors 13 and 14. The low-frequency generator 9 is based on a discharge capacitor 15, a first blocking inductor 16, a second blocking inductor 17 with a current-limiting resistor 18, and a variable resistor 19. Connected in parallel to the discharge capacitor 15 is an indicator lamp 20 with a resistor 21.

The discharge capacitor 15 is parallel-connected, on the one hand, through the variable resistor 19, to the high-voltage power supply source 8 and, on the other hand, through the inductors 16 and 17, to the resistor 18, i.e., to the gas-discharge tube 6.

The knob of the variable resistor 19 is mechanically coupled to a mains switch 22 and is brought to the front panel of the housing 1 as well as the indicator lamp 20.

In order to reduce the effect of the field electromagnetic component on the operation of the device, provision is therein made for parallel-connected a capacitor 23 (FIG.3) and an inductor 24, both being electrically isolated from the rest of the elements of the device and establishing an additional resonant LC-circuit which is interconnected with the grounding terminal 2 and the housing 1 at a single point whose position is chosen such that a maximum loop of the electromagnetic component of the energy field is on the external surface of the housing 1.

The capacitor 23 and the inductor 24 are tuned to resonance with the field electromagnetic component, whereby its adverse effect during operation of the device can be reduced.

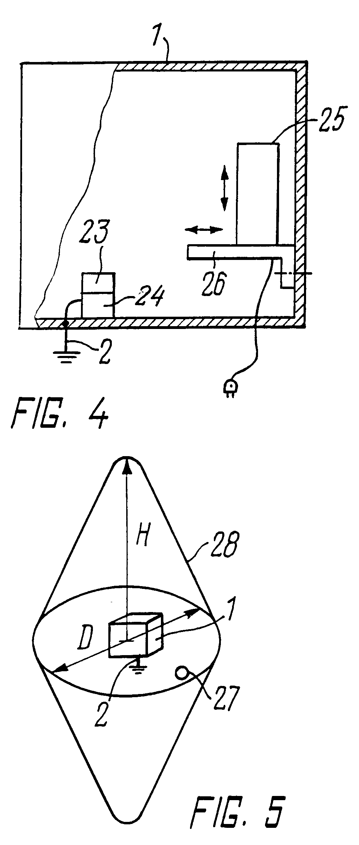

To provide a possibility of changing the shape of the energy field, the means 25 (FIG.4) for producing a high-frequency arc discharge is so accommodated in the housing 1 that a distance to its geometrical center is variable. To this end, a bracket 26 is fixed in position inside the housing 1 to carry the arc-discharge producing means 25 is fitted. An up-and-down arrow indicates schematically the direction of motion of the bracket 26 together with the means 25 mounted thereon during assembly and selection of working parameters, and a right-left arrow indicates the direction of motion of the means 25 itself over the surface of the bracket 26, carried out likewise prior to starting operation of the device.

FIG.5 shows schematically the appearance of a shielding energy field 28 established with the use of the proposed device, said field having the shape of an ellipsoidal solid of revolution whose major axis is oriented vertically. A biological object 27 is schematically shown in the effective zone of the energy field having a height 2H and a diameter D.

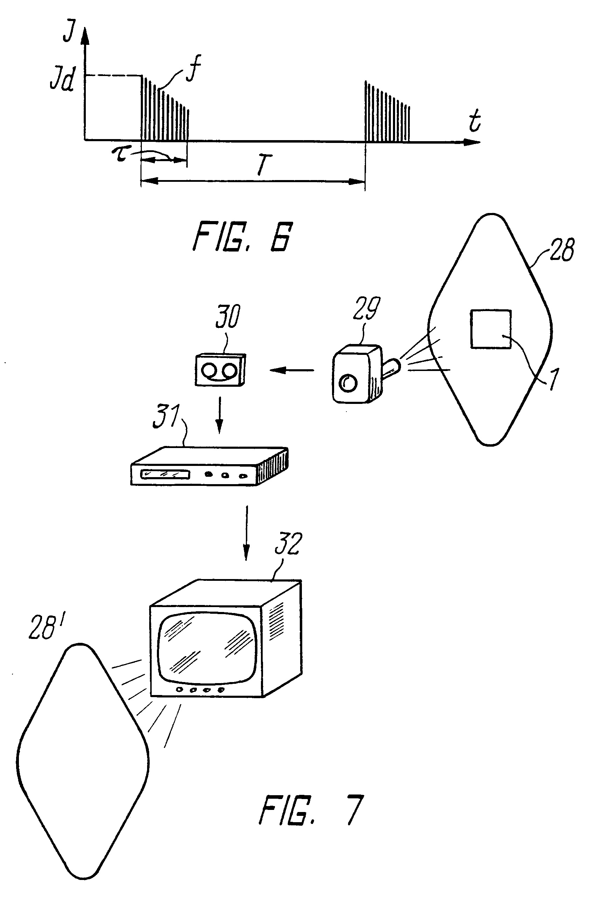

FIG.6 represents high-frequency pulses of an arc discharge, where current value is laid off as ordinate and time is laid off as abscissa. The symbols in FIG.6 are as follows: T is the charge-discharge time of the capacitor 15 (FIG.2), f is the oscillation frequency of the arc-discharge current, F is the time of a high-frequency arc discharge, and Id is maximum discharge current.

According to the invention proposed herein, provision is therein made for recording and transferring an information about the energy field 28. To this aim, the device is provided with recording and reproduction means. In a particular case shown in FIG.7, such means may be a video camera 29 with a magnetic information medium 30 (video cassette), a video tape recorder 31 and a television receiver 32 connected thereto and reproducing the recorded energy field 28'.

The shape, orientation, and properties of the reproduced energy field 28' are the same as in the original field 28.

In the general case there may be a number of the television receivers 32 reproducing the recorder energy field 28' either simultaneously or in succession.

The modern technical capabilities of telecommunication means allows one to combine information recording and reproducing in various combinations known commonly in the present state of the art; therefore, to promote understanding of the essence of the present invention, its embodiment considered hereinbelow presents one of the simplest technical solutions.

The herein-proposed method for energy protection of a biological object resides in that the energy field 28 (FIG.5) is established, concentrated in a confined space and possessing the properties of a biological-energy shield in the form of an ellipsoidal solid of revolution having its major axis vertically oriented.

Generation of the energy field 28 having the aforesaid shape and properties is ensured during operation of the present device for energy protection of a biological object, which functions as follows.

By rotating the knob of the variable resistor 19 (FIG.2) one causes the mains switch 22 to close, whereby A.C. mains voltage is applied to the primary of the transformer 10, the secondary voltage thereof is rectified by a voltage doubling circuit built around the diodes 11, 12 and the capacitors 13, 14. The resultant direct voltage charges the discharge capacitor 15 through the variable resistor 19. Once the voltage across the capacitor 15 has reached the value of the breakdown voltage of the gas-discharge tube, a breakdown occurs between the electrodes 7 (FIG.1), with the result that a high-frequency plasma arc discharge arises in the discharge gap between said electrodes, with an arc current oscillation frequency f (FIG.6) set by the resonant LC-circuit 3 (FIG.2). The time F (FIG.6) of the high-frequency arc discharge depends on the time for the capacitor 15 (FIG.2) to discharge down to the extinction voltage of the gas-discharge tube 6 (FIGS.1, 2).

The charge-discharge time T (FIG.6) of the capacitor 15 (FIG.2) is adjustable in accordance with the discharge recurrence frequency while the maximum discharge current Id (FIG.6) is limited by the resistor 18 (FIG.2).

Thus, the capacitor 15 periodically charges through the variable resistor 19 and discharges through the blocking inductors 16, 17, the resistor 18, and the gas-discharge tube 6, while the indicator lamp 20 blinks at a rate equal to the discharge recurrence frequency.

By changing the recurrence frequency of high-frequency discharges and the operating time of the entire device, one establishes the energy field 28 (FIG.5) having some dimensions and energy density or other and possessing the properties of a biological-energy shield.

The variable resistor 19 (FIG.2) is used to control and adjust the discharge recurrence frequency and hence the generation rate of the field 28, its dimensions and energy density which does not virtually reduce from the center of the field 28 towards its periphery.

The presence of the thus-generated energy field 28 and its cross-sectional energy density are monitored by diverse techniques. First and foremost, by using heretofore-known biological locators (frame, rod) which response to the presence of the field 28. Furthermore, the energy field 28 can be detected with the aid of neutral information media (distilled or dechlorinated water, physiological saline, and others), which are capable of changing their physicochemical properties (structure, electrical and heat conduction), which is registered with the use of known research equipment in the course of numerous experiments.

One more technique of detecting the energy field 28 is an extrasensory one, which corroborates completely the results of the techniques mentioned above.

The shielding energy field 28 possesses a characteristic aftereffect. Thus, the field 28 is dispersed slowly for 20-30 minutes after deenergizing the device rather than disappears instantaneously.

Protective properties of a biological-energy shield manifest themselves in that in case of effects produced by harmful external radiations close to the biological-energy shield as to their properties and structure, or in case of unauthorized extrasensory effects said biological-energy shield prevents said adverse factors from penetration in its own effective zone. The biological objects 27 exposed to the effect of the energy field 28 are relieved from adverse effects of geopathogenic factors and diverse unauthorized effects, extrasensory ones inclusive, as well as are put out of suggested and stress states.

A therapeutic effect resulting from exposure to the energy field 28 manifests itself in stimulated growth of tissues in case of affections of non-hereditary etiology, namely, healing of wounds, burns, certain kinds of ulcers, and non-infectious dermatitis; in stimulating the immune and hematopoietic systems in diseases of non-hereditary etiology; in intensifying and normalizing the metabolic processes at the cell level; in retarding or even suppressing the growth of certain kinds of tumors, malignant ones inclusive, and in stimulating sexual activity.

Apart from the aforementioned property, that is, protection of the biological objects 27 against unauthorized extrasensory effects, the field 28 possesses the property of "catching", in its effective zone, "foul" biological energy, transferring it towards the housing 1 of the device, and throwing into the earth through the grounding terminal 2. This feature of the biological shield can find application when the device is used for getting locations rid of "foul" biological energy, neutralizing geopathogenic zones, as well as for therapy of the biological objects 27. Thus, "foul" biological energy resulting from the effect of harmful external radiations, such as ionizing radiation, is permanently withdrawn by the field 28 from the biological object 27 and is thrown into the earth through the grounding terminal 2, thus rendering a therapeutic action to said object and adding to the concentration of the vital energy thereof.

It is noteworthy that the generated energy field 28 acts favorably on the sowing seeds of plants and the sperm of animals, thus increasing the germinating ability and productivity of seeds and promoting selection work.

There have been conducted experiments with donor sperm in animal husbandry. The vital capacity of the sperm remains unaffected after preservation and depreservation, and viability of the youngsters, poultry inclusive, increases.

Use of the energy field 28 in plant raising for treating sowing seeds increases germinating ability of seeds, as well as to increase crop productivity after prolonged preservation of sowing seeds, and to add to the keeping quality of farm products.

Thus, for instance, there have been carried out experiments on treating the seeds of spring barley which demonstrate a more than 50% rise of the seed germinating force, as well as experiments on treating diverse bacteria at the stage of active growth and fission which show cessation of growth of bacterial colonies and even degradation of some colonies, especially those of actinomycetes.

The device of the embodiment presented in FIG.3 operates as described above, the sole difference residing in that provision of a LC-circuit, consisting of the independent grounded capacitor 23 and the inductor 24 which are tuned to resonance with the frequency of the electromagnetic component enables one to reduce a negative effect of said field component on the operation of the device.

According to the embodiment of the device shown in FIG.4, the position of the means 25 for producing a high-frequency arc discharge in the housing 1 can be varied. In this case the geometrical parameters of the generated energy field 28 (FIG.5) are changed to suit the geometrical dimensions of the biological object 27. Thus, when the field 28 is to be more oblong, the means 25 is displaced upwards and towards the center of the housing 1, and when the field 28 is to be more flattened, downwards and away from the center of the housing 1. Such a change in the shape and geometrical dimensions of the field 28 is noted by the aforesaid monitoring means, (biological location, extrasensory technique, and others).

According to another embodiment of the proposed method for energy protection of a biological object, there is produced an energy field possessing the properties of an energy shield and shaped as an ellipsoidal solid of revolution having its major axis vertically oriented, the thus-established field is connected, through an optical transmission line, to an information medium, and recording said energy field is preceded by converting it into electromagnetic waves which are then transmitted, along electromagnetic communication lines, to an information medium with a possibility of repeated reproduction of said information for a biological object to be exposed to the effect thereof, the shape and properties of the reproduced energy field being similar to those of the originally established energy field.

An information about the energy field can be transcribed onto a similar or different information medium, though the properties of the reproduced field are somewhat weakened.

The presence of a reproduced energy field is detected by the aforementioned monitoring means.

The herein-proposed method is illustrated by the device for energy protection of a biological object an embodiment of which is presented in FIG.7. The generated energy field 28 is recorded onto the magnetic information medium 30 with the use of the video camera 29. Then the information about the energy field 28 is transmitted and reproduced, with the use of the video tape recorder 34 or directly from the video camera 29 (depending on its capabilities), to the television receiver 32.

As a result, the reproduced energy field 28' is established nearby the television receiver 32, having the same properties as in the field 28. Information can be transmitted to a number of the TV receivers 32 at a time.

According to another embodiment of the proposed method, exposed to the effect of the energy field can be the preliminarily produced image of a biological object rather than the object itself, because the effect produced on the image of a biological object is equivalent to that produced directly on the object itself.

The image of a biological object may be established in the form of electromagnetic waves which are transmitted, either directly or in recorded form, to the television receiver located in the effective zone of the energy field 28' (not shown in the Drawing).

Use can also be made of a photograph of the biological object as its image, which is placed in the effective zone of the field 28'.

The method disclosed herein extends the functional capabilities of the present invention by making possible recording and transferring information about the shielding energy field with retention of all of its properties and merits mentioned before.

The intensity of exposure to the effect of the energy field 28' can be selected depending on the required level of effect with a constant value of the parameters of the field 28 recorded on the medium 30.

This is effected due to a direct relationship between the ratio of the actual size of the image of the biological object 27 and the actual dimensions of the energy field 28. There is also a direct relationship between the intensity of the effect produced by the energy field and the period of time that has elapsed from the instant when the image of the biological object 27 represented on the receiver, is produced.

Recording and transferring information about the shielding properties of the energy field can be carried out by using readily available technical means, which renders the method extensibly applicable in practice.

To promote understanding of the essence and advantageous features of the present invention given below is a specific example of its practical application.

The device made in accordance with the method illustrated in FIGS.1 and 2 is used for generating an energy field.

The cube-shaped housing and the fastening element of the grounding terminal are made of aluminium, and the grounding terminal itself, of a copper wire conductor having a cross-sectional area of 6 sq.mm. The gas-discharge tube is filled with hydrogen. The diameter of the tube electrodes is 6 mm, the discharge gap therebetween is 5 mm, and the break-down voltage is from 0.9 to 1.1 kV.

The capacitor and the inductor establish a high-quality high-voltage resonance circuit having a resonant frequency of 16 MHz. The mains voltage is 220 V and the rectified voltage taken off the transformer secondary is 2.5 kV.

Duration (tau) of a high-frequency discharge is 4 µs and the discharge period T is within the adjustable range of from 0.1 to 0.01 s, which corresponds to a discharge recurrence frequency of 10-100 Hz. Maximum discharge current Id is from 400 to 600 A.

After a 100-second operating period of the device an ellipsoidal energy field is generated therearound, having a diameter D in the thickened zone from 30 to 40 m and a height H equal to 3 or 4D.

### Industrial Applicability

The present invention can find application in non-traditional medicine for equalizing the energy balance in biological objects. Moreover, the invention can be used in agriculture, namely, in plant raising and animal husbandry.

## Claims

1. A method for energy protection of a biological object residing in that an energy field is established, concentrated in a confined space, and a biological object (27) is exposed to the effect of said energy field, CHARACTERIZED in that the energy field (28) possesses the properties of a biological-energy shield and is shaped as an ellipsoidal solid of revolution having its major axis vertically oriented.

2. A method for energy protection of a biological object residing in that an energy field is established and transmitted, through an optical transmission line, to a recorder, whereupon the information thus recorded on an information medium is transmitted for the biological object (27) to be exposed to the effect thereof, CHARACTERIZED in that the energy field (28) possesses the properties of a biological energy shield in the form of an ellipsoidal solid of revolution having its major axis oriented vertically, and recording said energy field (28) is preceded by converting it into electromagnetic waves which are then transmitted along electromagnetic communication lines to an information medium (30) with a possibility of repeated reproduction of said information for the biological object (27) to be exposed to the effect thereof, the shape and properties of the reproduced energy field (28') being similar to those of the originally built up energy field (28).

3. A method according to Claim 2, CHARACTERIZED in that information in the form of electromagnetic waves is transmitted to at least one receiver (32).

4. A method according to Claim 2, or 3, CHARACTERIZED in that reproduction of said information is preceded by its transcribing to a similar or different information medium (30).

5. A method according to Claim 2, or 3, CHARACTERIZED in that an image of the biological object (27) is produced beforehand and is then exposed to the effect of the reproduced information, thus producing the effect of said information directly on the biological object (27).

6. A method according to Claim 5, CHARACTERIZED in that an image of the biological object (27) is generated in the form of electromagnetic waves which are transmitted to one of the receivers (32).

7. A method according to Claim 5, CHARACTERIZED in that an image of the biological object (27) is generated in the form of a photograph.

8. A method according to Claim 6, CHARACTERIZED in that when reproducing the information, the intensity of the effect produced by the energy field (28') on the image of the biological object (27) is chosen such that there is a direct relationship of said intensity to the period of time that has elapsed from the instant when the image of the biological object (27) represented on the receiver is produced.

9. A method according to Claim 5, or 6, or 7, CHARACTERIZED in that when reproducing the information, the intensity of the effect produced by the energy field (28) on the image of the biological object (27) is chosen such that a direct relationship of said intensity to the ratio between the actual size of the image of the biological object (27) and the actual dimensions of the energy field (28) is observed.

10. A device for energy protection of a biological object, comprising a non-magnetic hollow housing (1) appearing as a plane-base geometrical figure, CHARACTERIZED in that it comprises a means (25) for producing a high-frequency arc discharge, said means being accommodated in the housing (1) which is closed and is provided with a grounding terminal (2).

11. A device according to Claim 10, CHARACTERIZED in that the means (25) for producing a high-frequency arc discharge comprises a high-voltage power source (8), a low-frequency generator (9), a high-frequency arc-discharge generator, and a resonant LC-circuit (3), all of them being parallel-connected to one another.

12. A device according to Claim 10, CHARACTERIZED in that the high-frequency arc-discharge generator is built around a gas-discharge tube (6) having coaxial electrodes (7).

13. A device according to Claim 10, or 11, or 12, CHARACTERIZED in that it comprises a capacitor (23) and an inductor (24) which build up an additional LC-circuit electrically connected to the grounding terminal (2) and the housing (1) at a single point whose position is chosen such that a maximum loop of the electromagnetic component of the energy field (28) is over the external surface of the housing (1).

14. A device according to Claim 10, or 11, or 12, or 13, CHARACTERIZED in that the housing (1) is made from aluminium.

15. A device according to Claim 10, or 11, or 12, or 13, CHARACTERIZED in that the housing (1) is cube-shaped.

16. A device according to Claim 10, or 11, CHARACTERIZED in that the means (25) for producing a high-frequency arc discharge is enclosed in the housing (1) so that a distance to its geometrical center is variable.

17. A device according to any one of Claims 10 through 16, CHARACTERIZED in that it is equipped with a video camera (29) so positioned as to perform video-filming of the generated energy field (28), and with at least one television receiver (32) provided with a means (31) for reproducing the recorded information about the generated energy field (28).
